# EUROPEAN PATENT APPLICATION

(11) **EP 4 399 985 A1**
(43) Date of publication of application: **17.07.2024**
(21) Application number: 21967067.6
(22) Date of filing: 06.12.2021
(51) Int. Cl.: A24F 40/46

(54) **AEROSOL GENERATING SYSTEM**

(71) Applicant: Japan Tobacco Inc., Tokyo 105-6927 (JP)
(72) Inventor: YAMADA, Manabu, Tokyo 130-8603 (JP); INOUE, Yasunobu, Tokyo 130-8603 (JP)
(74) Representative: Hoffmann Eitle
(86) International application number: PCT/JP2021/044643
(87) International publication number: WO 2023/105560

(57) **Abstract**

[Problem] To provide a system in which freedom of design related to a heating unit can be improved.

[Solution] This aerosol generating system comprises: a tubular member that has an opening into which an aerosol generating base material containing an aerosol source can be inserted; and a heating unit. The tubular member has a bottom wall that closes at least a portion of an end section on the opposite side of the opening, and the heating unit is configured in a planar shape. A portion of the heating unit is disposed so as to follow an outer surface of the tubular member, and another portion of the heating unit is folded from the bottom wall of the tubular member in a direction away from the opening of the tubular member

## Description

### Technical Field

The present invention relates to an aerosol generation system.

### Background Art

Inhaler devices, such as an electronic tobacco and a nebulizer, that generate a material to be inhaled by a user have been widely popularized. For example, an inhaler device generates aerosol to which a flavor component has been imparted, by using an aerosol source for generating the aerosol, and a substrate containing a flavor source or the like for imparting the flavor component to the generated aerosol. A user can taste a flavor by inhaling the aerosol generated by the inhaler device and to which the flavor component has been imparted. Hereinafter, an action of a user inhaling aerosol is also referred to as a puff or a puff action.

Improving heating efficiency has been desired for inhaler devices of a type that generates aerosol by heating a substrate. For example, Patent Literature 1 below discloses a technology that improves heating efficiency by heating a substrate while pressing the substrate.

### Citation List

### Patent Literature

Patent Literature 1: WO 2021/172255 A1

### Summary of Invention

### Technical Problem

In PTL 1 above, a sheet-shaped heater is disposed without a gap at an outer surface of a side wall of a chamber that accommodates a substrate. Therefore, an electrode for applying electric current to the heater is connected to the heater at the side wall of the chamber, which limits design.

Thus, the present invention has been made in consideration of the above problem, and an object of the present invention is to provide a structure capable of improving flexibility in design relating to a heater.

### Solution to Problem

In order to solve the above problem, according to an aspect of the present invention, there is provided an aerosol generation system including: a tubular member that has an opening into which an aerosol-source material containing an aerosol source is insertable; and a heater, wherein the tubular member includes a bottom wall that closes at least a portion of an end portion on a side opposite to the opening, wherein the heater has a flat shape, wherein a portion of the heater is disposed along an outer surface of the tubular member, and wherein another portion of the heater is bent from the bottom wall of the tubular member in a direction away from the opening of the tubular member

The heater may have a heat-producing region and a non-heat-producing region, and a portion of the heater, the portion being bent in the direction away from the opening of the tubular member, may be the non-heat-producing region.

At an end portion of the tubular member far from the opening, a portion of the heater, the portion being bent in the direction away from the opening of the tubular member, may be connected to a power supply that applies electric current to the heater.

The bottom wall of the tubular member may be provided with a projection that protrudes from an outer surface of the bottom wall, the heater may have a hole, and the heater may be disposed around the tubular member in a state in which the projection of the tubular member passes through the hole of the heater.

The hole of the heater may circumscribe the projection of the tubular member

A shape of each of the hole of the heater and the projection of the tubular member in a plane orthogonal to a longitudinal direction of the tubular member may be a circle.

The heater may have a heat-producing region and a non-heat-producing region, and the hole of the heater may be surrounded by the non-heat-producing region.

A portion of the heater may be disposed along, in the outer surface of the bottom wall of the tubular member, a portion except the projection.

The heater may be bent along a boundary portion between an outer surface of the bottom wall of the tubular member and an outer surface of a side wall of the tubular member and is disposed along the outer surface of the bottom wall of the tubular member and the outer surface of the side wall of the tubular member.

The side wall of the tubular member may include a pressing portion having an inner surface and an outer surface that are flat, the pressing portion may press the aerosol-source material inserted into the tubular member, and the heater may be bent along a boundary portion between the outer surface of the bottom wall of the tubular member and the outer surface of the pressing portion of the tubular member and disposed along the outer surface of the bottom wall of the tubular member and the outer surface of the pressing portion of the tubular member

The tubular member may include the pressing portion that includes two or more pressing portions, and the heater may be bent along a boundary portion between the outer surface of the bottom wall of the tubular member and the outer surface of each of the two or more pressing portions of the tubular member and disposed along the outer surface of the bottom wall of the tubular member and the outer surface of each of the two pressing portions of the tubular member.

The heater may have a heat-producing region and a non-heat-producing region, and the heater may be bent at the non-heat-producing region.

The heater may be constituted by a conductive track that is disposed on an electrically insulating substrate that has a flat shape, the heater may have a heat-producing region and a non-heat-producing region, and electric resistance of the conductive track disposed in the heat-producing region may be higher than electric resistance of the conductive track disposed in the non-heat-producing region.

The non-heat-producing region of the heater may be disposed at the bottom wall of the tubular member and at a side of a side wall of the tubular member, the side being close to the bottom wall, and the heat-producing region of the heater may be disposed at a side of the side wall of the tubular member, the side being close to the opening.

The conductive track may constitute a parallel circuit in the heat-producing region of the heater.

The conductive track may turn back at an end portion of the heat-producing region of the heater, the end portion being on a side far from the non-heat-producing region.

The heater may be constituted by a conductive track that is disposed on an electrically insulating substrate that has a flat shape, the heater may have a heat-producing region and a non-heat-producing region, the conductive track disposed in the heat-producing region may be made of SUS, the conductive track disposed in the non-heat-producing region may be made of a material that contains at least either one of copper and nickel, and the electrically insulating substrate may be made of polyimide.

The aerosol generation system may further include a heat transfer layer that has predetermined thermal conductivity, and the heat transfer layer may be wound to cover at least partially the tubular member and the heater that is disposed along an outer surface of a side wall of the tubular member.

The heat transfer layer may be made of graphite.

The aerosol generation system may further include the aerosol-source material.

### Advantageous Effects of Invention

As described above, according to the present invention, a structure enabling appropriate arrangement of a heater is provided.

### Brief Description of Drawings

[Fig. 1] Fig. 1 is a schematic diagram of an inhaler device according to a configuration example.
[Fig. 2] Fig. 2 is a schematic diagram of a physical configuration of an inhaler device according to the present embodiment.
[Fig. 3] Fig. 3 is a perspective view of a heater assembly illustrated in Fig. 2.
[Fig. 4] Fig. 4 is a perspective view of a chamber
[Fig. 5] Fig. 5 is a sectional view of the chamber as viewed in arrow 4-4 direction illustrated in Fig. 4.
[Fig. 6] Fig. 6 is a sectional view of the chamber as viewed in arrow 5-5 direction illustrated in Fig. 5.
[Fig. 7] Fig. 7 is a longitudinal sectional view of a chamber including a non-pressing portion in a state in which a stick substrate is held by a holder.
[Fig. 8] Fig. 8 is a longitudinal sectional view of a chamber including a pressing portion in a state in which a stick substrate is held by a holder.
[Fig. 9] Fig. 9 is a sectional view of the chamber as viewed in arrow 7-7 direction illustrated in Fig. 8.
[Fig. 10] Fig. 10 is a diagram of a configuration of a heater according to the present embodiment in plan view.
[Fig. 11] Fig. 11 is a perspective view of a state before the heater according to the present embodiment is disposed around a chamber
[Fig. 12] Fig. 12 is a perspective view of a state after the heater according to the present embodiment is disposed around the chamber
[Fig. 13] Fig. 13 is a bottom view of a state after the heater according to the present embodiment is disposed around the chamber
[Fig. 14] Fig. 14 is a perspective view of a heater assembly according to a modification.

### Description of Embodiments

Hereinafter, a suitable embodiment of the present invention will be described in detail with reference to the accompanying drawing. Note that structural elements having substantially the same functional configuration are given the same sign in the present description and the drawings to thereby omit duplicate description thereof.

### <1. Configuration example of inhaler device>

An inhaler device generates a material to be inhaled by a user. In the example described below, the material generated by the inhaler device is an aerosol. Alternatively, the material generated by the inhaler device may be gas.

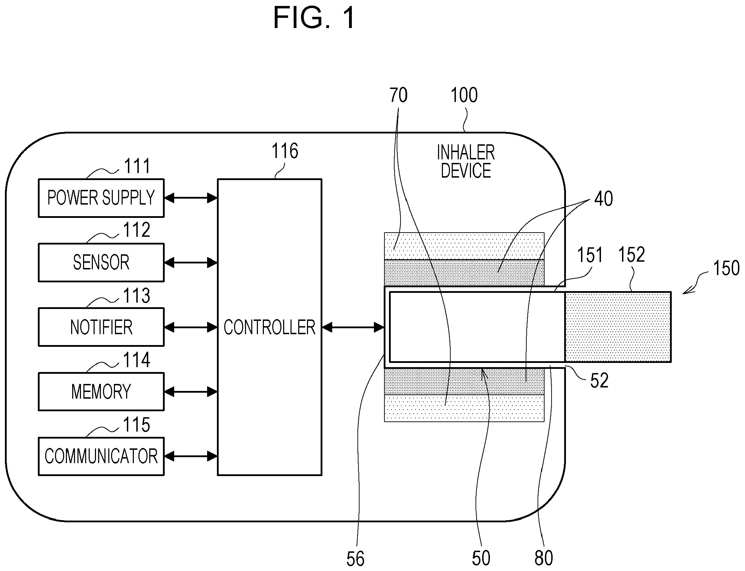

Fig. 1 is a schematic diagram of the inhaler device according to the configuration example. As illustrated in Fig. 1, an inhaler device 100 according to the present configuration example includes a power supply 111, a sensor 112, a notifier 113, a memory 114, a communicator 115, a controller 116, a heater 40, a chamber 50, and a heat insulator 70.

The power supply 111 stores electric power. The power supply 111 supplies electric power to the structural elements of the inhaler device 100 under the control of the controller 116. The power supply 111 may be a rechargeable battery such as a lithium ion secondary battery.

The sensor 112 acquires various items of information regarding the inhaler device 100. In an example, the sensor 112 may be a pressure sensor such as a condenser microphone, a flow sensor, or a temperature sensor, and acquire a value generated in accordance with the user's inhalation. In another example, the sensor 112 may be an input device that receives information input by the user, such as a button or a switch.

The notifier 113 provides information to the user. The notifier 113 may be a light-emitting device that emits light, a display device that displays an image, a sound output device that outputs sound, or a vibration device that vibrates.

The memory 114 stores various items of information for operation of the inhaler device 100. The memory 114 may be a non-volatile storage medium such as flash memory.

The communicator 115 is a communication interface capable of communication in conformity with any wired or wireless communication standard. Such a communication standard may be, for example, Wi-Fi (registered trademark) or Bluetooth (registered trademark).

The controller 116 functions as an arithmetic processing unit and a control circuit, and controls the overall operations of the inhaler device 100 in accordance with various programs. The controller 116 includes an electronic circuit such as a central processing unit (CPU) or a microprocessor, for example.

The chamber 50 accommodates and holds a stick substrate 150. The chamber 50 has an opening 52 that allows an internal space 80 formed in the inhaler device 100 to communicate with an external space. The stick substrate 150 is insertable through the opening 52 into the internal space 80 of the chamber 50. The chamber 50 accommodates the stick substrate 150 inserted through the opening 52 into the internal space 80.

The stick substrate 150 includes a substrate 151 and an inhalation port 152. The substrate 151 includes an aerosol source. The aerosol source is atomized to generate an aerosol. The aerosol source is a liquid such as polyhydric alcohol or water. Examples of the polyhydric alcohol include glycerine and propylene glycol. The aerosol source may include a flavor component that is either derived from tobacco or not derived from tobacco. For the inhaler device 100 that is a medical inhaler such as a nebulizer, the aerosol source may include a medicine. Note that the aerosol source is not limited to a liquid and may be a solid. The stick substrate 150 held by the chamber 50 includes the substrate 151 at least partially accommodated in the internal space 80 and the inhalation port 152 at least partially protruding from the opening 52. When the user inhales with the inhalation port 152 protruding from the opening 52 in his/her mouth, an aerosol generated from the substrate 151 reaches the inside of the mouth of the user.

The heater 40 heats the aerosol source to atomize the aerosol source and generate the aerosol. In an example, the heater 40 has a film-like shape and surrounds the outer circumference of the chamber 50. Subsequently, heat produced from the heater 40 heats the substrate 151 of the stick substrate 150 from the outer circumference, generating the aerosol. The heater 40 produces heat when supplied with electric power from the power supply 111.

The heat insulator 70 prevents heat from transferring from the heater 40 to the other structural elements. For example, the heat insulator 70 may be a vacuum heat insulator or an aerogel heat insulator.

The configuration example of the inhaler device 100 has been described above.

The stick substrate 150 is one example of an aerosol-source material containing an aerosol source. The inhaler device 100 and the stick substrate 150 work together to generate aerosol that is to be inhaled by a user. Therefore, the combination of the inhaler device 100 and the stick substrate 150 may be regarded as an aerosol generation system.

### <2. Technical features>

### (1) Configuration that heats a substrate while pressing the substrate

The inhaler device 100 according to the present embodiment has a configuration that heats the stick substrate 150 while pressing the stick substrate 150. Hereinafter, such a configuration will be described in detail.

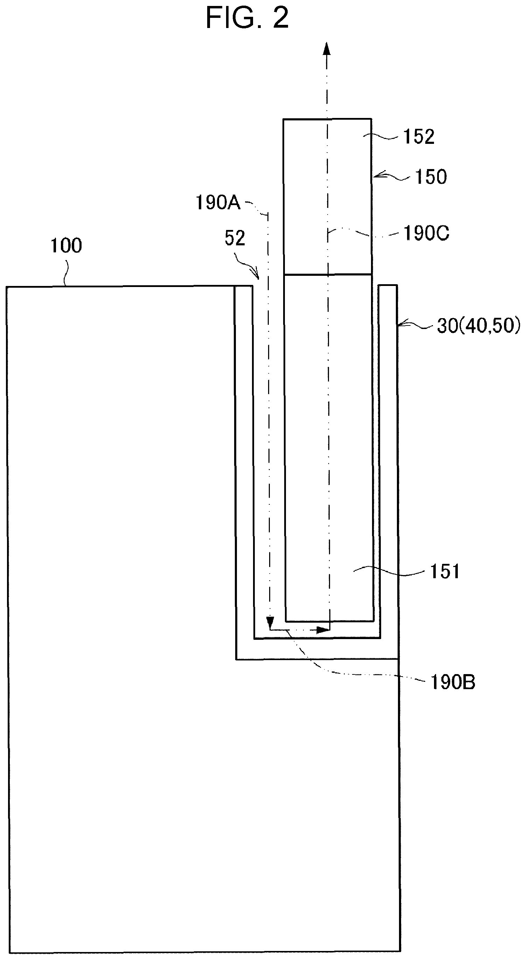

Fig. 2 is a schematic diagram of a physical configuration of the inhaler device 100 according to the present embodiment. As illustrated in Fig. 2, the inhaler device 100 includes a heater assembly 30 that includes the heater 40 and the chamber 50. As illustrated in Fig. 2, a gap is present between the heater assembly 30 and the stick substrate 150 in a state in which the stick substrate 150 is accommodated in the heater assembly 30 (more specifically, the chamber 50). When a user holds the stick substrate 150 in the mouth and inhales, the air that has flowed in through the opening 52 flows into the inside of the stick substrate 150 from the distal end of the substrate 151 via the gap and flows out from the tail end of the inhalation port 152 to the inside of the mouth of the user. That is, the air inhaled by the user flows in the order of an air flow 190A, an airflow 190B, and an airflow 190C and, in a state of being mixed with the aerosol generated from the stick substrate 150, is guided into the oral cavity of the user.

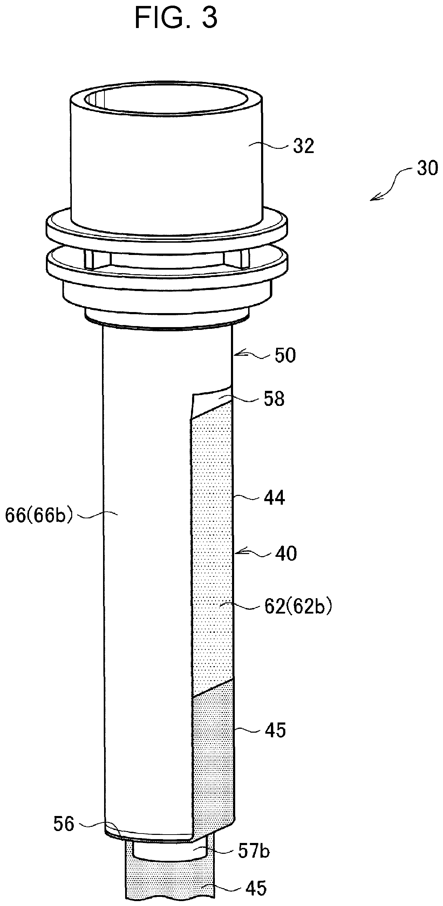

Fig. 3 is a perspective view of the heater assembly 30 illustrated in Fig. 2. As illustrated in Fig. 3, the heater assembly 30 includes a top cap 32, the heater 40, and the chamber 50. The heater 40 is disposed around the chamber 50. Thus, the heater 40 is configured to heat the stick substrate 150 received by the chamber 50. The top cap 32 may be configured to have a function of a guide for inserting the stick substrate 150 into the chamber 50 and to fix the chamber 50 to the inhaler device 100.

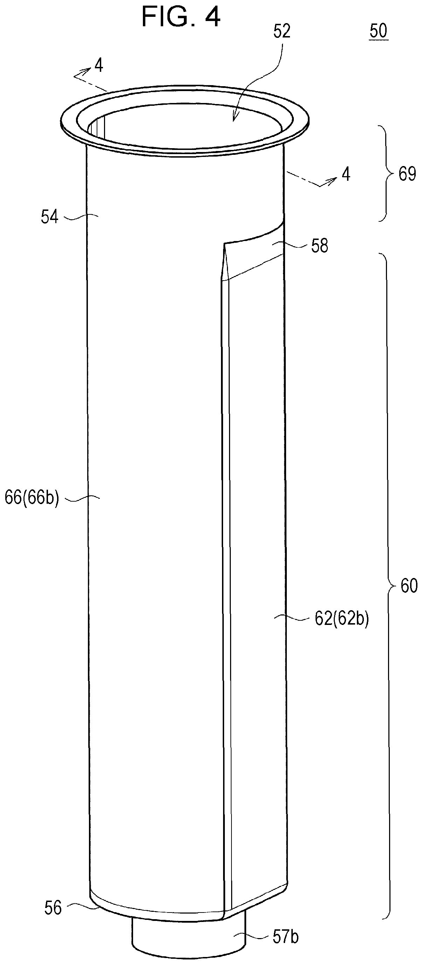

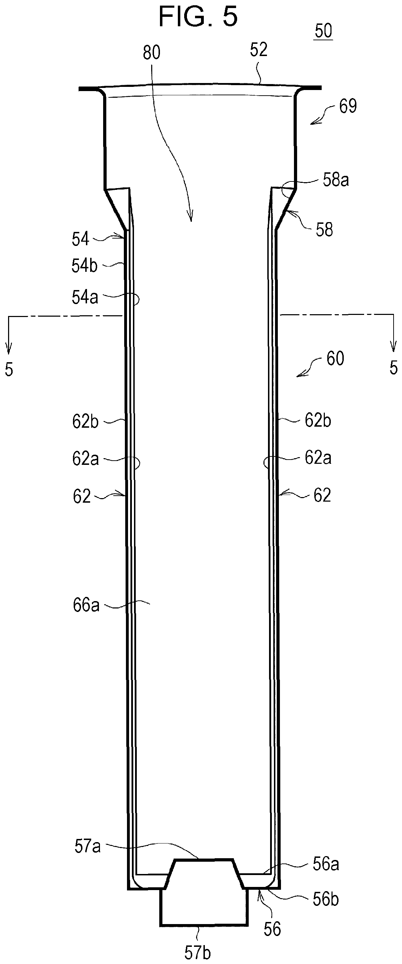

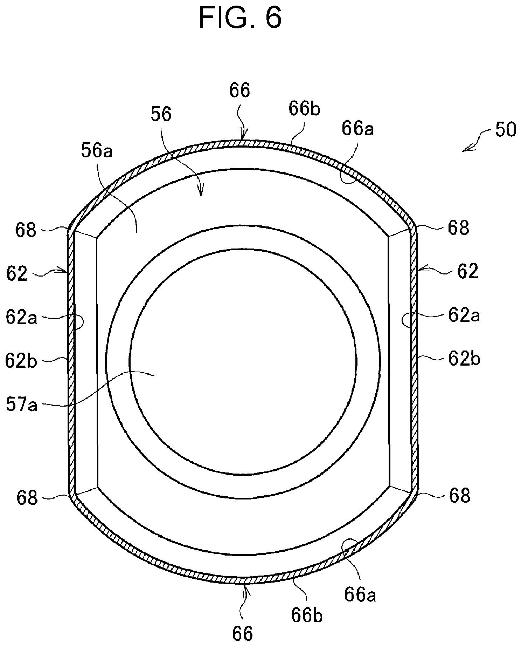

Fig. 4 is a perspective view of the chamber 50. Fig. 5 is a sectional view of the chamber 50 as viewed in arrow 4-4 direction illustrated in Fig. 4. Fig. 6 is a sectional view of the chamber 50 as viewed in arrow 5-5 direction illustrated in Fig. 5. As illustrated in Fig. 4 and Fig. 5, the chamber 50 is a bottomed tubular member that includes the opening 52, a side wall 54, and a bottom wall 56 that closes an end portion on the side opposite to the opening 52. The side wall 54 has an inner surface 54a and an outer surface 54b. The bottom wall 56 has an inner surface 56a and an outer surface 56b. The stick substrate 150 is inserted through the opening 52 into the chamber 50 and accommodated in the internal space 80 surrounded by the side wall 54 and the bottom wall 56. The chamber 50 is preferably made of a metal having high thermal conductivity and may be made of, for example, stainless steel or the like. Consequently, the stick substrate 150 can be efficiently heated.

As illustrated in Fig. 4 and Fig. 5, the chamber 50 includes a holder 60 that holds the stick substrate 150. As illustrated in Fig. 5 and Fig. 6, the holder 60 includes a pressing portion 62 that presses a portion of the stick substrate 150, and a non-pressing portion 66. The pressing portion 62 has an inner surface 62a and an outer surface 62b. The non-pressing portion 66 has an inner surface 66a and an outer surface 66b. Each of the pressing portion 62 and the non-pressing portion 66 is a portion of the side wall 54 of the chamber 50.

Preferably, the opening 52 of the chamber 50 is capable of receiving the stick substrate 150 without pressing the stick substrate 150. The shape of the opening 52 of the chamber 50 in a plane orthogonal to the longitudinal direction (in other words, the direction in which the stick substrate 150 is inserted into the chamber 50 or the direction in which the side wall 54 of the chamber 50 extends) of the chamber 50 may be a polygonal shape or an elliptical shape. However, the shape is preferably a circular shape.

As illustrated in Fig. 4, Fig. 5, and Fig. 6, the chamber 50 in the present embodiment includes two or more pressing portions 62 in the circumferential direction of the chamber 50. As illustrated in Fig. 5 and Fig. 6, the two pressing portions 62 of the holder 60 face each other. The distance between the inner surfaces 62a of the two pressing portions 62 is preferably smaller at at least a portion than the width of a section of the stick substrate 150 disposed between the pressing portions 62, the stick substrate 150 being inserted into the chamber 50. As illustrated, the inner surface 62a of the pressing portion 62 is flat. The outer surface 62b of the pressing portion 62 is also flat. More simply, the pressing portion 62 is a portion formed as a flat plate in the side wall 54.

As illustrated in Fig. 6, the inner surface 62a of the pressing portion 62 has a pair of flat press surfaces facing each other and each having a flat shape. On the other hand, the inner surface 66a of the non-pressing portion 66 connects both ends of the pair of the flat press surfaces and has a pair of curved non-press surfaces facing each other and each having a curved shape. As illustrated, the non-press surfaces may each have a generally arc-shaped cross-section in a plane orthogonal to the longitudinal direction of the chamber 50. The outer surface 62b of the pressing portion 62 and the outer surface 66b of the non-pressing portion 66 are connected to each other with an angle therebetween, and a boundary 68 may be formed between the outer surface 62b of the pressing portion 62 and the outer surface 66b of the non-pressing portion 66. As illustrated in Fig. 6, the pressing portion 62 and the non-pressing portion 66 (that is, the side wall 54 of the chamber 50) may each have a uniform thickness.

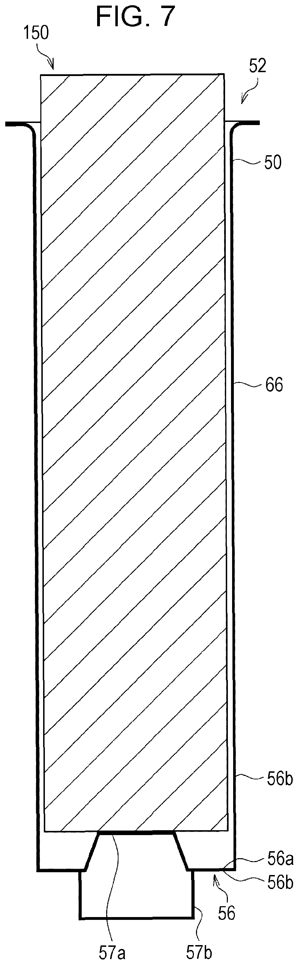

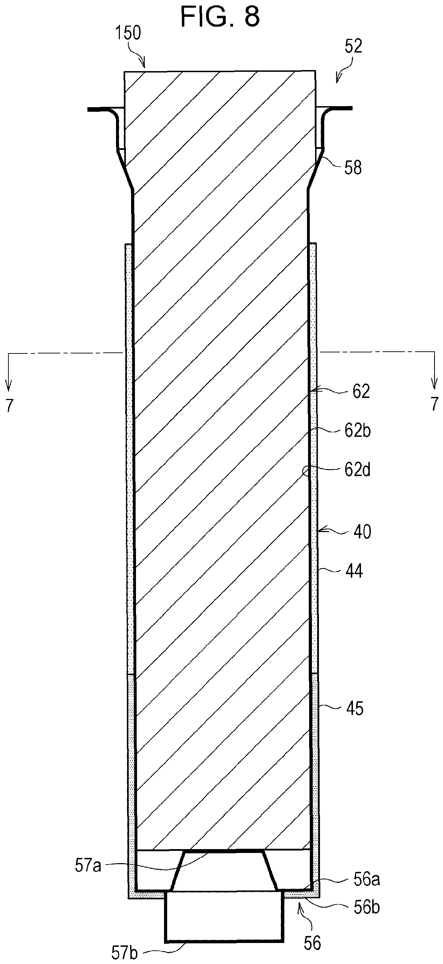

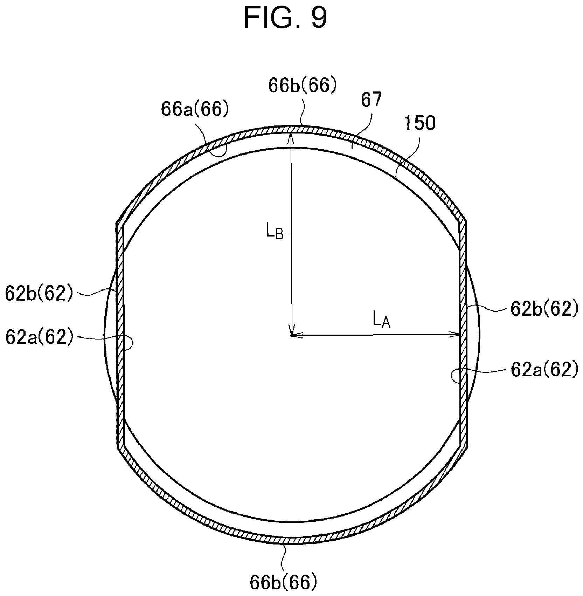

Fig. 7 is a longitudinal sectional view of the chamber 50 including the non-pressing portion 66 in a state in which the stick substrate 150 is held by the holder 60. Fig. 8 is a longitudinal sectional view of the chamber 50 including the pressing portion 62 in a state in which the stick substrate 150 is held by the holder 60. Fig. 9 is a sectional view of the chamber 50 as viewed in arrow 7-7 direction illustrated in Fig. 8. Note that a cross-section of the stick substrate 150 in a state before being pressed is illustrated in Fig. 9 for easy understanding of pressing of the stick substrate 150 by the pressing portion 62.

A gap 67, illustrated in Fig. 9, between the inner surface 66a of the non-pressing portion 66 and the stick substrate 150 is substantially maintained even when the stick substrate 150 is held by the holder 60 and when the stick substrate 150 is pressed and deformed by the pressing portion 62. This gap 67 may communicate with the opening 52 of the chamber 50 and an end surface (an end surface on the lower side in Fig. 7 and Fig. 8, that is, an end surface of the substrate 151 illustrated in Fig. 2) of the stick substrate 150 positioned in the chamber 50. It can be said that this gap 67 communicates with the opening 52 of the chamber 50 and an end surface (an end surface on the lower side in Fig. 7 and Fig. 8, that is, an end surface of the substrate 151 illustrated in Fig. 2) of the stick substrate 150, the end surface being positioned in the chamber 50 and being positioned far from the opening 52 of the chamber 50. Consequently, a flow path for air via the gap 67 and the inside of the stick substrate 150 is formed to extend from the opening 52 of the chamber 50 to an end surface (an end surface on the upper side in Fig. 7 and Fig. 8, that is, an end surface of the inhalation port 152 illustrated in Fig. 2) of the stick substrate 150 positioned outside the chamber 50. This eliminates the need to separately provide the inhaler device 100 with a flow path for guiding air that is to be supplied the stick substrate 150 and thus can simplify the structure of the inhaler device 100. In addition, since a section of the non-pressing portion 66 forming a portion of the gap 67 is exposed, the flow path can be easily cleaned. Further, since air is heated while the air passes through the gap 67, heating efficiency can be increased by effectively using heat radiation of the heater 40, and the temperature of the stick substrate 150 can be prevented from being excessively decreased by the air that flows in with a puff. As a result, power consumption of the heater 40 can be suppressed, and the flavor can be prevented from decreasing due to a temperature decrease of the stick substrate 150 with a puff. From the point of view of airflow resistance and the like, the height of the gap 67 between the inner surface 66a of the non-pressing portion 66 and the stick substrate 150 is preferably 0.1 mm or more and 1.0 mm or less, more preferably 0.2 mm or more and 0.8 mm or less, and most preferably 0.3 mm or more and 0.5 mm or less.

As illustrated in Fig. 9, in the state in which the stick substrate 150 is held by the holder 60, a distance L_{A} between the inner surface 62a of the pressing portion 62 and the center of the stick substrate 150 is shorter than a distance L_{B} between the inner surface 66a of the non-pressing portion 66 and the center of the stick substrate 150. Such a configuration can shorten the distance between the heater 40 disposed at the outer surface 62b of the pressing portion 62 and the center of the stick substrate 150, compared with a case where the pressing portion 62 is not provided. Thus, efficiency in heating of the stick substrate 150 can be increased.

As illustrated in Fig. 5 to Fig. 8, the bottom wall 56 of the chamber 50 is provided with a first projection 57a protruding from the inner surface 56a of the bottom wall 56. The first projection 57a has, for example, a frustoconical shape whose top surface is flat. The top surface of the first projection 57a is formed to be smaller than at least an end surface of the stick substrate 150. Consequently, as illustrated in Fig. 7 and Fig. 8, the bottom wall 56 supports a portion of the stick substrate 150 inserted into the chamber 50 by the first projection 57a such that at least a portion of the end surface of the stick substrate 150 is exposed. In addition, the bottom wall 56 may also support a portion of the stick substrate 150 by the first projection 57a such that the exposed end surface of the stick substrate 150 communicates with the gap 67.

As illustrated in Fig. 5 to Fig. 8, the bottom wall 56 of the chamber 50 is provided with a second projection 57b that protrudes from the outer surface 56b of the bottom wall 56. The second projection 57b has, for example, a cylindrical shape whose top surface is flat. Then, the second projection 57b is disposed at a central portion of the bottom wall 56 of the chamber 50.

As illustrated in Fig. 6 and Fig. 9, the inner surface 66a of the non-pressing portion 66 of the holder 60 is curved in a plane orthogonal to the longitudinal direction of the chamber 50. The shape of the inner surface 66a of the non-pressing portion 66 in a plane orthogonal to the longitudinal direction of the chamber 50 is preferably the same at any position in the longitudinal direction of the chamber 50 as the shape of the opening 52 in a plane orthogonal to the longitudinal direction of the chamber 50. In other words, preferably, the inner surface 66a of the non-pressing portion 66 is formed by extending the inner surface, which forms the opening 52, of the chamber 50 in the longitudinal direction.

As illustrated in Fig. 3 to Fig. 5, preferably, the chamber 50 includes a tubular non-holder 69 between the opening 52 and the holder 60. The non-holder 69 is a portion of the chamber 50, the portion not contributing to holding of the stick substrate 150. For example, the non-holder 69 may be formed to be larger than the stick substrate 150 in a plane orthogonal to the longitudinal direction of the chamber 50. Consequently, a space may be formed between the non-holder 69 and the stick substrate 150 in a state in which the stick substrate 150 is held by the holder 60.

As illustrated in Fig. 5 to Fig. 9, preferably, the outer peripheral surface of the holder 60 has the same shape and the same size (the outer circumferential length of the holder 60 in a plane orthogonal to the longitudinal direction of the holder 60) over the entire length in the longitudinal direction of the holder 60.

Further, as illustrated in Fig. 4 and Fig. 5, preferably, the chamber 50 includes a first guide 58 that has a tapered surface 58a connecting the inner surface, which forms the opening 52, of the chamber 50 (that is, the non-holder 69) and the inner surface 62a of the pressing portion 62 to each other. Due to the pressing portion 62 and the non-holder 69 being connected to each other smoothly by the first guide 58, the stick substrate 150 can be suitably guided to the holder 60 in the process of insertion of the stick substrate 150 into the chamber 50.

As illustrated in Fig. 3, the heater 40 is disposed around the chamber 50. Thus, when the heater 40 produces heat, the chamber 50 is heated from the outer circumference thereof, and the stick substrate 150 is heated by the heat transferred from the chamber 50. Consequently, aerosol can be generated from the stick substrate 150.

As illustrated in Fig. 3, the heater 40 is disposed at the outer surface 62b of the pressing portion 62. Preferably, the heater 40 is disposed at the outer surface 62b of the pressing portion 62 without a space therebetween. In addition, preferably, the heater 40 is disposed over the entirety of the outer surface 62b of the pressing portion 62. Note that, preferably, the heater 40 is disposed not to extend beyond the outer surface 62b of the pressing portion 62. Naturally, the heater 40 may be disposed to extend beyond the outer surface 62b of the pressing portion 62 to the outer surface 66b of the non-pressing portion 66.

As illustrated in Fig. 3, the heater 40 has a heat-producing region 44 and a non-heat-producing region 45. The heat-producing region 44 is a region that produces heat when electric current is applied to the heater 40. The non-heat-producing region 45 is a region that does not produce heat or produces heat minimally even when electric current is applied to the heater 40. The heat-producing region 44 is disposed at the outer surface 62b of the pressing portion 62. Such a configuration makes it possible to efficiently heat the stick substrate 150 while pressing the stick substrate 150 by the pressing portion 62.

As described above, the inhaler device 100 according to the present embodiment heats the stick substrate 150 while pressing and holding the stick substrate 150 by the pressing portion 62. Such a configuration exerts various effects described below.

First, thermal conductivity from the heater 40 to the stick substrate 150 is improved. That is, efficiency in heating of the stick substrate 150 can be improved. Since efficiency in heating of the stick substrate 150 is improved, the temperature of the stick substrate 150 can be caused to reach a target temperature quickly. Thus, a time required for preheating (heating from a start of heating until a puff is enabled) can be shorten. Further, since efficiency in heating of the stick substrate 150 is improved, followability of the temperature of the stick substrate 150 with respect to a temperature change of the heater 40 can be improved. As a result, firstly, the generation amount of the aerosol can be more easily controlled. Secondary, even when the temperature of the stick substrate 150 is decreased due to a puff by a user, the temperature of the stick substrate 150 can be immediately returned to its original temperature. Thirdly, the influence of external environment, such as outside temperature, can be reduced.

In addition, the inhaler device 100 according to the present embodiment heats the stick substrate 150 from the outer circumference while pressing the stick substrate 150. Such a configuration can implement, regardless of the shape of the aerosol source in the stick substrate 150, the above-described improvement in the efficiency in heating of the stick substrate 150 and the above-described improvement in the followability of the temperature of the stick substrate 150. Further, such a configuration can implement, regardless of an error of the shape or the size of the stick substrate 150 due to a variation generated in the manufacturing step of the stick substrate 150, the above-described improvement in the efficiency in heating of the stick substrate 150 and the above-described improvement in the followability of the temperature of the stick substrate 150. In contrast, it may be difficult to exert these effects in a comparative example that employs a configuration in which a blade-shaped heater is inserted into the stick substrate 150 and in which the stick substrate 150 is heated from the inside thereof. This is because it may be difficult in the comparative example to cause the blade-shaped heater and an aerosol source in the stick substrate 150 to come into contact with each other appropriately even if the stick substrate 150 is pressed from the outer circumference thereof.

In addition, in the inhaler device 100 according to the present embodiment, the heat-producing region 44 of the heater 40 is disposed at the pressing portion 62 that presses the stick substrate 150. Therefore, the inhaler device 100 according to the present embodiment heats the stick substrate 150 at the pressing portion 62. Such a configuration can improve heating efficiency compared with a comparative example in which the heat-producing region 44 of the heater 40 is disposed at not only the pressing portion 62 but also the non-pressing portion 66 and in which the stick substrate 150 is heated from the whole circumference thereof. This is because the area of the heat-producing region 44 can be reduced, and watt density can be increased.

Note that the heat insulator 70 may be disposed so as to surround the heater assembly 30 from the outer circumference thereof in the inhaler device 100 according to the present embodiment. In such a case, the thickness of an air layer formed between the outer surface 62b of the pressing portion 62 and the inner surface of the heat insulator 70 can be increased due to the outer surface 62b of the pressing portion 62 being positioned closer than the outer surface 66b of the non-pressing portion 66 to the center of the internal space 80. That is, the thickness of the heat insulator 70 overlapped by the pressing portion 62 can be increased. Therefore, a heat insulating effect of the heat insulator 70 can be improved.

### (2) Configuration of preventing positional displacement of the heater 40

The inhaler device 100 according to the present embodiment has a configuration that prevents positional displacement of the heater 40. Hereinafter, the configuration that prevents positional displacement of the heater 40 will be described in detail.

Here, the positional displacement is displacement between an ideal arrangement of the heater 40 and an actual arrangement of the heater 40. The positional displacement includes two types of positional displacement such as positional displacement in manufacture and positional displacement in usage. The positional displacement in manufacture is positional displacement that is generated in disposing of the heater 40 around the chamber 50. The positional displacement in usage is positional displacement that is generated in the process of using the manufactured inhaler device 100. Hereinafter, the positional displacement refers to both the positional displacement in manufacture and the positional displacement in usage unless specifically mentioned. Note that the ideal arrangement of the heater 40 in the present embodiment refers to arrangement in which, as illustrated in Fig. 3, the heat-producing region 44 of the heater 40 is disposed at the outer surface 62b of the pressing portion 62 of the chamber 50.

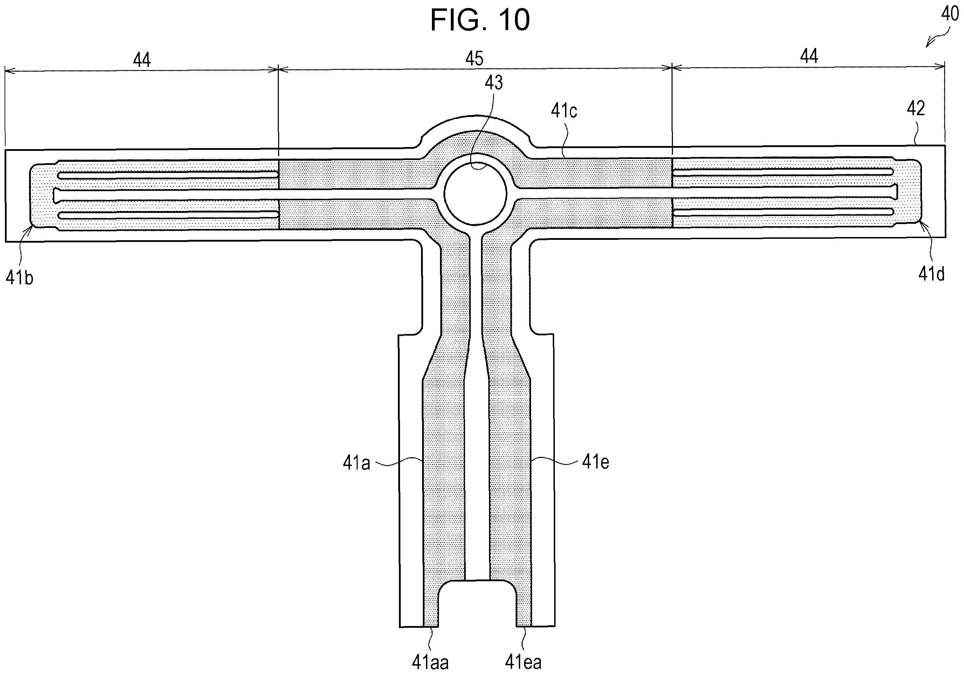

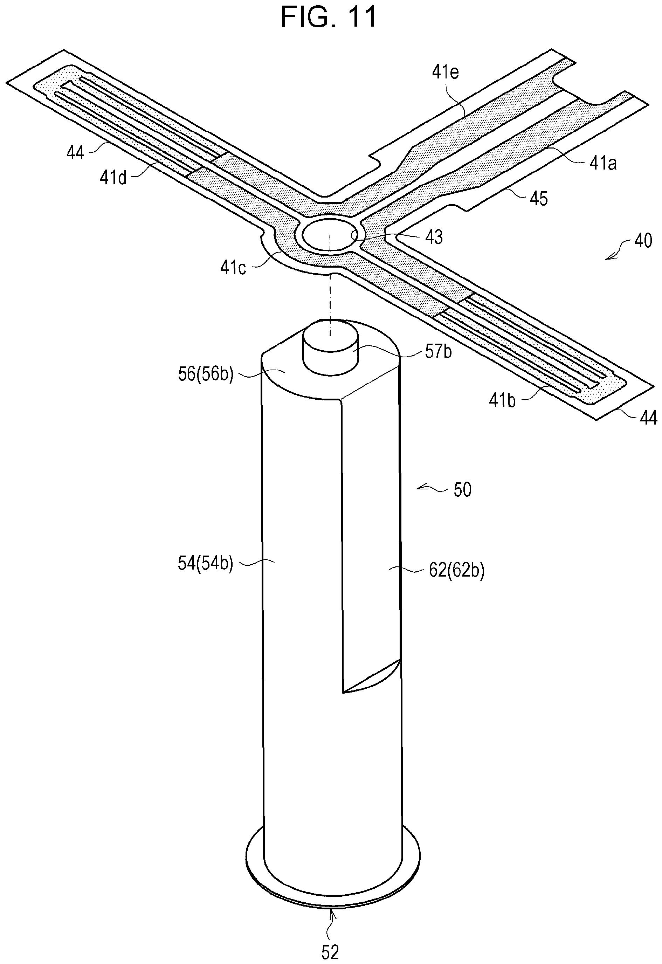

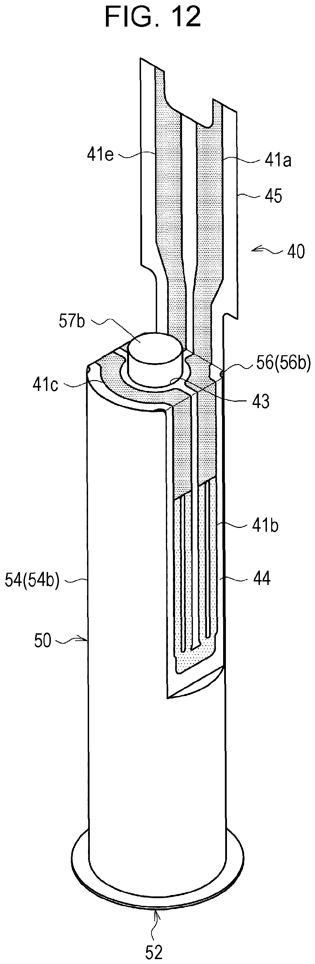

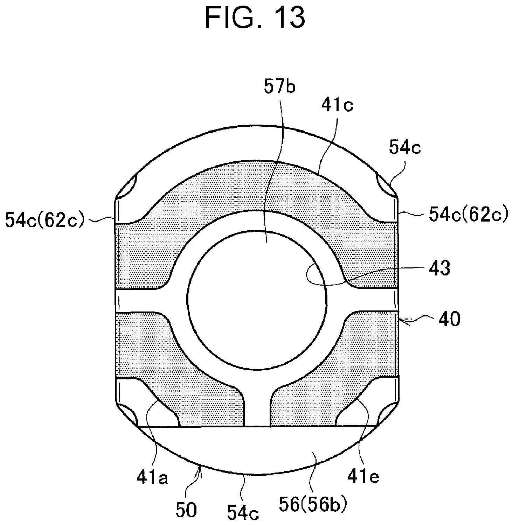

Fig. 10 is a diagram of a configuration of the heater 40 according to the present embodiment in plan view. Fig. 11 is a perspective view of a state before the heater 40 according to the present embodiment is disposed around the chamber 50. Fig. 12 is a perspective view of a state after the heater 40 according to the present embodiment is disposed around the chamber 50. Fig. 13 is a bottom view of a state after the heater 40 according to the present embodiment is disposed around the chamber 50.

As illustrated in Fig. 10, the heater 40 has a flat shape. Then, as illustrated in Fig. 11 and Fig. 12, the heater 40 is bent to extend along the outer surface of the chamber 50 and is disposed along the outer surface of the chamber 50.

As illustrated in Fig. 10, the heater 40 has a T-shape in plan view in a state before being bent. Then, as illustrated in Fig. 11 and Fig. 12, a horizontal bar portion of the T-shape of the heater 40 is bent to extend along the outer surface of the chamber 50 and is disposed along the outer surface of the chamber 50. On the other hand, as illustrated in Fig. 12, a vertical bar portion of the T-shape of the heater 40 is bent in a direction opposite to the horizontal bar portion of the T-shape and is separated from the outer surface of the chamber 50.

As illustrated in Fig. 10, the heater 40 has a hole 43. More specifically, the hole 43 is provided in a central portion of the T-shape in a state before being bent.

As illustrated in Fig. 10, the heater 40 may be constituted by a conductive track 41 (41a to 41e) that is disposed on an electrically insulating substrate 42 that has a flat shape. The conductive track 41 is a circuit that is made of an electrically conductive material. The electrically insulating substrate 42 is a substrate that is made of an electrically insulative material. As an example of the electrically insulative material, polyimide can be presented. For example, the heater 40 may be a film heater that is constituted by a conductive track sandwiched by two polyimide films. As other examples of the electrically insulative material, PET (Polyethylene terephthalate), a fluororesin, and the like can be presented.

As illustrated in Fig. 10, the heater 40 has the heat-producing region 44 and the non-heat-producing region 45. The heat-producing region 44 is a region that produces heat when electric current is applied to the heater 40. The non-heat-producing region 45 is a region that does not produce heat or produces heat minimally even when electric current is applied to the heater 40. That is, the electric resistance of the conductive track 41 (41b, 41d) disposed in the heat-producing region 44 is higher than the electric resistance of the conductive track 41 (41a, 41c, 41e) disposed in the non-heat-producing region 45. As one example, as illustrated in Fig. 10, the conductive track 41 disposed in the heat-producing region 44 may be formed to be thin while the conductive track 41 disposed in the non-heat-producing region 45 is formed to be wide. Consequently, the above-described magnitude relationship of the electric resistance can be implemented. In addition, the conductive track 41 disposed in the heat-producing region 44 may be made of, for example, SUS (steel use stainless). On the other hand, the conductive track 41 disposed in the non-heat-producing region 45 may be made of, for example, a material containing at least either one of copper and nickel. Specifically, the conductive track 41 disposed in the non-heat-producing region 45 may be made of SUS that is plated with copper and nickel. In such a case, for example, the SUS may have a thickness of 30 µm, the nickel may have a thickness of 30 µm, and the copper may have a thickness of 5 µm. Such a configuration also can implement the above-described magnitude relationship of the electric resistance and can increase heat resistance of the conductive track 41 in the heat-producing region 44. Naturally, the material of the conductive track 41 is not limited to the examples described above and may be another material, such as aluminum.

As illustrated in Fig. 10, the conductive track 41 may constitute a parallel circuit in the heat-producing region 44 of the heater 40. For example, the conductive track 41b constitutes, at each of two locations before and after turning back at an end portion of the heat-producing region 44, a parallel circuit in which two paths are arranged parallel to each other. Similarly, the conductive track 41d constitutes, at each of two locations before and after turning back at an end portion of the heat-producing region 44, a parallel circuit in which two paths are arranged parallel to each other. Naturally, the number of the paths arranged parallel to each other is not limited to two and may be three or more, and the number of the paths arranged parallel to each other may differ between before and after turning back. Such a configuration can reduce deviation in heat distribution in the heat-producing region 44. In addition, the conductive track 41 may constitute a parallel circuit also in the non-heat-producing region 45 of the heater 40. That is, each of the conductive tracks 41a, 41c, and 41e may constitute a parallel circuit. As the number of the parallel paths increases, the width of one conductive track decreases, and the non-heat-producing region 45 can be thus more easily bent.

As illustrated in Fig. 10, the conductive track 41b turns back, in the heat-producing region 44 of the heater 40, at an end portion on a side far from the non-heat-producing region 45 and is connected to each of the conductive track 41a and the conductive track 41c that are disposed around the hole 43. Similarly, the conductive track 41d turns back, in the heat-producing region 44 of the heater 40, at an end portion on a side far from the non-heat-producing region 45 and is connected to each of the conductive track 41e and the conductive track 41c that are disposed around the hole 43. As described above, the conductive track 41 is disposed to extend from the lower end of the vertical bar portion of the T-shape of the heater 40, bypass the hole 43, make a round at the horizontal bar portion of the T-shape, and return to the lower end of the vertical bar portion of the T-shape again.

As illustrated in Fig. 11, Fig. 12, and Fig. 13, the heater 40 is disposed around the chamber 50 in a state in which the second projection 57b provided on the bottom wall 56 of the chamber 50 passes through the hole 43 of the heater 40. With such a configuration, positional displacement that may be generated in the heater 40 can be limited to be within the range of a space between the second projection 57b provided on the bottom wall 56 of the chamber 50 and the hole 43 of the heater 40. Therefore, positional displacement of the heater 40 can be reduced.

As illustrated in Fig. 11, Fig. 12, and Fig. 13, the hole 43 of the heater 40 circumscribes the second projection 57b of the chamber 50. Such a configuration can eliminate the space between the second projection 57b provided on the bottom wall 56 of the chamber 50 and the hole 43 of the heater 40. Consequently, the range of positional displacement that may be generated in the heater 40 can be minimized as much as possible. That is, positional displacement of the heater 40 can be prevented.

As illustrated in Fig. 13, the shape of each of the hole 43 of the heater 40 and the second projection 57b of the chamber 50 may be a circle in a plane orthogonal to the longitudinal direction of the chamber 50. Such a configuration can easily cause the hole 43 of the heater 40 to circumscribe the second projection 57b of the chamber 50. In addition, with such a configuration, positioning for causing the arrangement of the heater 40 to be an ideal arrangement may be easily performed in the process of manufacture by rotating the heater 40 with the second projection 57b as the axis of the rotation in a state in which the second projection 57b of the chamber 50 passes through the hole 43 of the heater 40. Naturally, the cross-sectional shape of each of the hole 43 of the heater 40 and the second projection 57b of the chamber 50 may be any shape, such as a polygonal shape or an elliptical shape. As one example, the cross-sectional shape of each of the hole 43 of the heater 40 and the second projection 57b of the chamber 50 may be a shape formed by two equal-length parallel lines that are connected to each other at respective two ends by two circular arcs, similarly to the shape that is formed by the two pressing portions 62 and the two non-pressing portions 66 illustrated in Fig. 6. As another example, the cross-sectional shape of each of the hole 43 of the heater 40 and the second projection 57b of the chamber 50 may be a shape formed by one straight line whose two ends are connected to each other by one circular arc, that is, a shape of a segment of a circle.

As illustrated in Fig. 13, a portion of the heater 40 is disposed along, in the outer surface 56b of the bottom wall 56 of the chamber 50, a portion except the second projection 57b. With such configuration, the position of the heater 40 can be limited such that the portion of the heater 40 surrounding the hole 43 and, in the outer surface 56b of the bottom wall 56 of the chamber 50 , the portion except the second projection 57b are in contact (for example, close contact) with each other. Therefore, positional displacement of the heater 40 in the longitudinal direction of the chamber 50 can be prevented.

As illustrated in Fig. 11 and Fig. 12, a portion of the heater 40 extending beyond the bottom wall 56 is bent. Specifically, a portion (the horizontal bar portion of the T-shape) of the heater 40 is bent in a direction toward the side wall 54 of the chamber 50. As a result, the portion (the horizontal bar portion of the T-shape) of the heater 40 is disposed along the outer surface of the chamber 50. On the other hand, the other portion (the vertical bar portion of the T-shape) of the heater 40 is bent from the bottom wall 56 of the chamber 50 in a direction away from the opening 52 of the chamber 50. Such a configuration eliminates the need to dispose the entirety of the heater 40 to extend along the outer surface of the chamber 50. Consequently, flexibility in design relating to the heater 40 can be improved.

As illustrated in Fig. 11 and Fig. 12, the non-heat-producing region 45 of the heater 40 is bent. Such a configuration can prevent a bent position from receiving a load generated by heat production. Therefore, possibility of occurrence of malfunction of the heater 40 can be reduced compared with a case where the heat-producing region 44 of the heater 40 is bent.

As illustrated in Fig. 10 and Fig. 12, the portion of the heater 40 bent in the direction away from the opening 52 of the chamber 50 is the non-heat-producing region 45. Specifically, a portion of the non-heat-producing region 45 where the conductive track 41a and the conductive track 41e are disposed is bent in the direction away from the opening 52 of the chamber 50. Such a configuration can prevent heat from transferring to components (for example, the power supply 111 and the like) other than the chamber 50.

At an end portion of the chamber 50 far from the opening 52, the portion of the heater 40 bent in the direction away from the opening 52 of the chamber 50 is connected to the power supply 111. Specifically, an end portion 41aa of the conductive track 41a disposed in the non-heat-producing region 45, the end portion 41aa being present on the side bent in the direction away from the opening 52 of the chamber 50, is connected to the power supply 111. Similarly, an end portion 41ea of the conductive track 41e disposed in the non-heat-producing region 45, the end portion 41ea being present on the side bent in the direction away from the opening 52 of the chamber 50, is connected to the power supply 111. Such a configuration enables electric power supply to the heater 40 while heat transfer to the power supply 111 is prevented.

As illustrated in Fig. 10, the hole 43 of the heater 40 is provided at a position surrounded by the non-heat-producing region 45. Then, as illustrated in Fig. 12, the non-heat-producing region 45 of the heater 40 is disposed at the bottom wall 56 of the chamber 50 and at a side of the side wall 54 close to the bottom wall 56. On the other hand, at a side of the side wall 54 of the chamber 50 close to the opening 52, the heat-producing region 44 of the heater 40 is disposed. Consequently, the heat-producing region 44 is disposed at a position corresponding to a central portion of the stick substrate 150 except a tip portion (that is, a side close to the bottom wall 56) of the stick substrate 150 accommodated in the chamber 50. Such a configuration can reduce the area of the heat-producing region 44, compared with a case where the heat-producing region 44 is disposed at a position corresponding to not only the central portion of the stick substrate 150 but also a position corresponding to the tip portion. As a result, the watt density is increased, and it is thus possible to efficiently heat the stick substrate 150. In addition, by avoiding heating of the tip portion of the stick substrate 150, the aerosol can be prevented from leaking out to the outside from the tip portion of the stick substrate 150. As a result, a decrease in the amount of the aerosol that is sent to reach a user can be prevented, and stain of the inner surface of the chamber 50 can be also prevented.

As illustrated in Fig. 11, Fig. 12, and Fig. 13, the heater 40 is bent along a boundary portion 54c between the outer surface 56b of the bottom wall 56 of the chamber 50 and the outer surface 54b of the side wall 54 and is disposed along the outer surface 56b of the bottom wall 56 of the chamber 50 and the outer surface 54b of the side wall 54. With such a configuration, it is possible to easily dispose the heater 40 to extend along the outer surface (the outer surface 56b and the outer surface 54b) of the chamber 50 by disposing first the heater 40 along the outer surface 56b of the bottom wall 56 and then bending the heater 40 along the boundary portion 54c. In addition, the heater 40 can be fixed in a state in which a fold of the heater 40 and the boundary portion 54c are aligned. Consequently, positional displacement of the heater 40 can be prevented.

In particular, as illustrated in Fig. 11, Fig. 12, and Fig. 13, the heater 40 is bent along a boundary portion 62c between the outer surface 56b of the bottom wall 56 of the chamber 50 and the outer surface 62b of the pressing portion 62 of the chamber 50 and is disposed along the outer surface 56b of the bottom wall 56 of the chamber 50 and the outer surface 62b of the pressing portion 62 of the chamber 50. Since both the outer surface 56b of the bottom wall 56 and the outer surface 62b of the pressing portion 62 are flat, the boundary portion 62c is linear. Therefore, the heater 40 can be fixed in a state in which the linear fold of the heater 40 and the linear boundary portion 62c coincide with each other. Further, in the outer surface (the outer surface 56b and the outer surface 54b) of the chamber 50, each of the outer surface 56b of the bottom wall 56 and the outer surface 62b of the pressing portion 62 at which the heater 40 is disposed is flat. Therefore, the heater 40 formed to be flat can be disposed at the outer surface of the chamber 50 without a space therebetween. As described above, positional displacement of the heater 40 can be prevented.

More specifically, as illustrated in Fig. 11, Fig. 12, and Fig. 13, the heater 40 is bent along the boundary portion 62c between the outer surface 56b of the bottom wall 56 of the chamber 50 and the outer surface 62b of each of the two pressing portions 62 of the chamber 50 and is disposed along the outer surface 56b of the bottom wall 56 of the chamber 50 and the outer surface 62b of each of the two pressing portions 62 of the chamber 50. Here, the two pressing portions 62 are provided at positions facing each other, and the horizontal bar portion of the T-shape of the heater 40 is disposed along the outer surface 62b of each of these two pressing portions 62 facing each other. With such a configuration, the heater 40 is fixed to sandwich and hold the chamber 50 from the outer side of the pressing portions 62 that face each other. Consequently, positional displacement of the heater 40 can be prevented.

### <3. Supplement>

A suitable embodiment of the present invention has been described above with reference to the accompanying drawings. The present invention is, however, not limited to such an example. It is obvious that a person having general knowledge in the technical field that the present invention belongs can conceive various modifications or corrections within the scope of the technical idea described in the claims, and it should be naturally understood that these modifications and corrections also belong to the technical scope of the present invention.

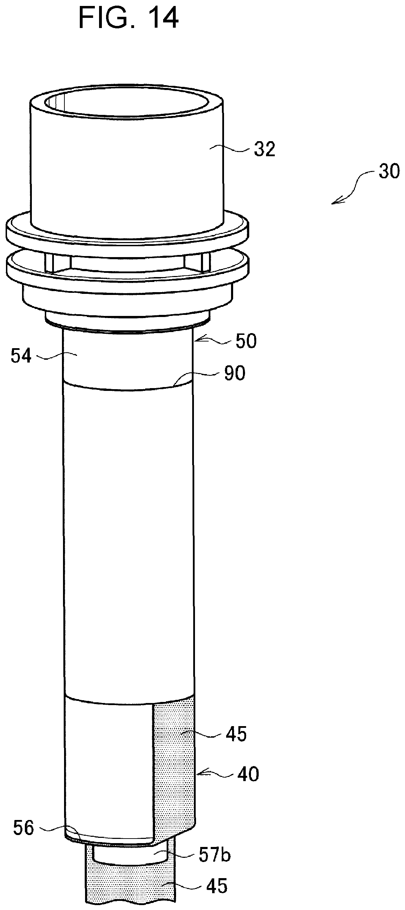

The inhaler device 100 may further include a configuration for further improving the efficiency in heating by the heater 40. This will be described in detail with reference to Fig. 14. Fig. 14 is a perspective view of the heater assembly 30 according to a modification. As illustrated in Fig. 14, a heat transfer layer 90 may be wound to cover at least partially the chamber 50 and the heater 40 disposed along the outer surface 54b of the side wall 54 of the chamber 50. In particular, the heat transfer layer 90 desirably covers the entirety of the heat-producing region 44. The heat transfer layer 90 is a sheet-shaped member having predetermined thermal conductivity. The thermal conductivity of the heat transfer layer 90 is desirably higher than at least the thermal conductivity of the chamber 50. As one example, the heat transfer layer 90 may be made of graphite. As another example, the heat transfer layer 90 may be made of aluminum, copper, or the like. With such a configuration, a portion of the chamber 50 where the heater 40 is not disposed can be heated through the heat transfer layer 90, and it is thus possible to improve the efficiency in heating of the stick substrate 150.

In the aforementioned embodiment, an example in which the heater 40 is configured as a film heater including the conductive track 41 sandwiched by the two electrically insulating substrates 42 has been described. The present invention is, however, not limited to such an example. For example, as an alternative to the conductive track 41, a conductive film may be used. The conductive film is a film having conductivity and may be formed by, for example, depositing ITO (Indium Tin Oxide).

In the aforementioned embodiment, an example in which the bottom wall 56 of the chamber 50 is provided with one second projection 57b has been described. The present invention is, however, not limited to such an example. The bottom wall 56 of the chamber 50 may be provided with two or more second projections 57b. In such a case, it is sufficient for the heater 40 to have the same number of the holes 43 as the number of the second projections 57b.

In the aforementioned embodiment, an example in which the chamber 50 includes a pair of the pressing portions 62 that face each other has been described. The present invention is, however, not limited to such an example. The pair of pressing portions 62 do not necessarily face each other. The chamber 50 may include one pressing portion 62 and may include three or more pressing portions 62. In such a case, it is sufficient for the heater 40 to be disposed along at least one pressing portion 62. That is, the heater 40 is not limited to have a T-shape in plan view in a state before being bent. It is sufficient for the heater 40 to have a shape corresponding to the relative position of the pressing portion 62 with respect to the bottom wall 56 of the chamber 50 in plan view in a state before being bent.

In the aforementioned embodiment, an example in which the chamber 50 is configured as a cylinder substantially has been described. The present invention is, however, not limited to such an example. The chamber 50 may be configured as an elliptical cylinder substantially and may be configured to have a rectangular cylinder shape.

In the aforementioned embodiment, an example in which the bottom wall 56 of the chamber 50 completely closes the end portion of the chamber 50 on the side opposite to the opening 52 has been described. The present invention is, however, not limited to such an example. It is sufficient for the bottom wall 56 of the chamber 50 to at least partially close the end portion of the chamber 50 on the side opposite to the opening 52. That is, the bottom wall 56 of the chamber 50 may have a hole. For example, a hole extending through the first projection 57a and the second projection 57b may be provided, and an airflow path through which air is introduced into the internal space 80 of the chamber 50 may be connected to the hole.

In the aforementioned embodiment, an example in which the heater 40 has the hole 43 has been described. The present invention is, however, not limited to such an example. It is sufficient for the heater 40 to be provided with a section corresponding to the second projection 57b provided at the bottom wall 56 of the chamber 50. Then, it is sufficient for the heater 40 to be disposed around the chamber 50 in a state in which the second projection 57b of the chamber 50 coincides with the section of the heater 40 corresponding to the second projection 57b of the chamber 50. For example, a central portion of the T-shape of the heater 40 in plan view before being bent may be provided with a notch having a size that contains the hole 43. Then, the heater 40 may be disposed around the chamber 50 in a state in which the notch circumscribes the second projection 57b.

In the aforementioned embodiment, an example in which the bottom wall 56 is provided with the second projection 57b has been described. The present invention is, however, not limited to such an example. It is sufficient for the bottom wall 56 to be provided with an element that corresponds to the hole 43 and that serves as a mark for preventing positional displacement of the heater 40. For example, on the bottom wall 56, a circular mark corresponding to the hole 43 may be drawn, or a recess that corresponds to the hole 43 may be provided.

Note that the following configurations also belong to the technical scope of the present invention.
(1) An aerosol generation system including:
   a tubular member that has an opening into which an aerosol-source material containing an aerosol source is insertable; and
   a heater, wherein
   the tubular member includes a bottom wall that closes at least a portion of an end portion on a side opposite to the opening,
   the heater has a flat shape,
   a portion of the heater is disposed along an outer surface of the tubular member, and
   another portion of the heater is bent from the bottom wall of the tubular member in a direction away from the opening of the tubular member.
(2) The aerosol generation system according to (1) above, wherein
   the heater has a heat-producing region and a non-heat-producing region, and
   a portion of the heater, the portion being bent in the direction away from the opening of the tubular member, is the non-heat-producing region.
(3) The aerosol generation system according to (1) or (2) above, wherein
   at an end portion of the tubular member far from the opening, a portion of the heater, the portion being bent in the direction away from the opening of the tubular member, is connected to a power supply that applies electric current to the heater.
(4) The aerosol generation system according to any one of (1) to (3) above, wherein
   the bottom wall of the tubular member is provided with a projection that protrudes from an outer surface of the bottom wall,
   the heater has a hole, and
   the heater is disposed around the tubular member in a state in which the projection of the tubular member passes through the hole of the heater.
(5) The aerosol generation system according to (4) above, wherein
   the hole of the heater circumscribes the projection of the tubular member
(6) The aerosol generation system according to (4) or (5) above, wherein
   a shape of each of the hole of the heater and the projection of the tubular member in a plane orthogonal to a longitudinal direction of the tubular member is a circle.
(7) The aerosol generation system according to any one of (4) to (6) above, wherein
   the heater has a heat-producing region and a non-heat-producing region, and
   the hole of the heater is surrounded by the non-heat-producing region.
(8) The aerosol generation system according to any one of (4) to (7) above, wherein a portion of the heater is disposed along, in the outer surface of the bottom wall of the tubular member, a portion except the projection.
(9) The aerosol generation system according to any one of (1) to (8) above, wherein
   the heater is bent along a boundary portion between an outer surface of the bottom wall of the tubular member and an outer surface of a side wall of the tubular member and is disposed along the outer surface of the bottom wall of the tubular member and the outer surface of the side wall of the tubular member.
(10) The aerosol generation system according to (9) above, wherein
   the side wall of the tubular member includes a pressing portion having an inner surface and an outer surface that are flat,
   the pressing portion presses the aerosol-source material inserted into the tubular member, and
   the heater is bent along a boundary portion between the outer surface of the bottom wall of the tubular member and the outer surface of the pressing portion of the tubular member and is disposed along the outer surface of the bottom wall of the tubular member and the outer surface of the pressing portion of the tubular member
(11) The aerosol generation system according to (10) above, wherein
   the tubular member includes the pressing portion that includes two or more pressing portions, and
   the heater is bent along a boundary portion between the outer surface of the bottom wall of the tubular member and the outer surface of each of the two or more pressing portions of the tubular member and is disposed along the outer surface of the bottom wall of the tubular member and the outer surface of each of the two pressing portions of the tubular member
(12) The aerosol generation system according to any one of (9) to (11) above, wherein
   the heater has a heat-producing region and a non-heat-producing region, and
   the heater is bent at the non-heat-producing region.
(13) The aerosol generation system according to any one of (1) to (12) above, wherein
   the heater is constituted by a conductive track that is disposed on an electrically insulating substrate that has a flat shape,
   the heater has a heat-producing region and a non-heat-producing region, and
   electric resistance of the conductive track disposed in the heat-producing region is higher than electric resistance of the conductive track disposed in the non-heat-producing region.
(14) The aerosol generation system according to (13) above, wherein the non-heat-producing region of the heater is disposed at the bottom wall of the tubular member and at a side of a side wall of the tubular member, the side being close to the bottom wall, and
   the heat-producing region of the heater is disposed at a side of the side wall of the tubular member, the side being close to the opening.
(15) The aerosol generation system according to (13) or (14) above, wherein
   the conductive track constitutes a parallel circuit in the heat-producing region of the heater.
(16) The aerosol generation system according to any one of (13) to (15) above, wherein
   the conductive track turns back at an end portion of the heat-producing region of the heater, the end portion being on a side far from the non-heat-producing region.
(17) The aerosol generation system according to any one of (1) to (16) above, wherein
   the heater is constituted by a conductive track that is disposed on an electrically insulating substrate that has a flat shape,
   the heater has a heat-producing region and a non-heat-producing region,
   the conductive track disposed in the heat-producing region is made of SUS,
   the conductive track disposed in the non-heat-producing region is made of a material that contains at least either one of copper and nickel, and
   in which the electrically insulating substrate is made of polyimide.
(18) The aerosol generation system according to any one of (1) to (17) above, wherein
   the aerosol generation system further comprises a heat transfer layer that has predetermined thermal conductivity, and
   the heat transfer layer is wound to cover at least partially the tubular member and the heater that is disposed along an outer surface of a side wall of the tubular member.
(19) The aerosol generation system according to (18) above, wherein
   the heat transfer layer is made of graphite.
(20) The aerosol generation system according to any one of (1) to (19) above, wherein
   the aerosol generation system further comprises the aerosol-source material.

### Reference Signs List

- 100: inhaler device
- 111: power supply
- 112: sensor
- 113: notifier
- 114: memory
- 115: communicator
- 116: controller
- 150: stick substrate
- 151: substrate
- 152: inhalation port
- 30: heater assembly
- 32: top cap
- 40: heater
- 41: conductive track
- 42: electrically insulating substrate
- 43: hole
- 44: heat-producing region
- 45: non-heat-producing region
- 50: chamber
- 52: opening
- 54: side wall (54a: inner surface, 54b: outer surface, 54c: boundary portion)
- 56: bottom wall (56a: inner surface, 56b: outer surface)
- 57a: first projection
- 57b: second projection
- 58: first guide
- 58a: tapered surface
- 60: holder
- 62: pressing portion (62a: inner surface, 62b: outer surface, 62c: boundary portion)
- 66: non-pressing portion (66a: inner surface, 66b: outer surface)
- 67: gap
- 68: boundary
- 69: non-holder
- 70: heat insulator
- 80: internal space
- 90: heat transfer layer

## Claims

1. An aerosol generation system comprising:
a tubular member that has an opening into which an aerosol-source material containing an aerosol source is insertable; and
a heater, wherein
the tubular member includes a bottom wall that closes at least a portion of an end portion on a side opposite to the opening,
the heater has a flat shape,
a portion of the heater is disposed along an outer surface of the tubular member, and
another portion of the heater is bent from the bottom wall of the tubular member in a direction away from the opening of the tubular member.

2. The aerosol generation system according to claim 1, wherein
the heater has a heat-producing region and a non-heat-producing region, and
a portion of the heater, the portion being bent in the direction away from the opening of the tubular member, is the non-heat-producing region.

3. The aerosol generation system according to claim 1 or 2, wherein
at an end portion of the tubular member far from the opening, a portion of the heater, the portion being bent in the direction away from the opening of the tubular member, is connected to a power supply that applies electric current to the heater.

4. The aerosol generation system according to any one of claims 1 to 3, wherein
the bottom wall of the tubular member is provided with a projection that protrudes from an outer surface of the bottom wall,
the heater has a hole, and
the heater is disposed around the tubular member in a state in which the projection of the tubular member passes through the hole of the heater.

5. The aerosol generation system according to claim 4, wherein
the hole of the heater circumscribes the projection of the tubular member.

6. The aerosol generation system according to claim 4 or 5, wherein
a shape of each of the hole of the heater and the projection of the tubular member in a plane orthogonal to a longitudinal direction of the tubular member is a circle.

7. The aerosol generation system according to any one of claims 4 to 6, wherein
the heater has a heat-producing region and a non-heat-producing region, and
the hole of the heater is surrounded by the non-heat-producing region.

8. The aerosol generation system according to any one of claims 4 to 7, wherein
a portion of the heater is disposed along, in the outer surface of the bottom wall of the tubular member, a portion except the projection.

9. The aerosol generation system according to any one of claims 1 to 8, wherein
the heater is bent along a boundary portion between an outer surface of the bottom wall of the tubular member and an outer surface of a side wall of the tubular member and is disposed along the outer surface of the bottom wall of the tubular member and the outer surface of the side wall of the tubular member.

10. The aerosol generation system according to claim 9, wherein
the side wall of the tubular member includes a pressing portion having an inner surface and an outer surface that are flat,
the pressing portion presses the aerosol-source material inserted into the tubular member, and
the heater is bent along a boundary portion between the outer surface of the bottom wall of the tubular member and the outer surface of the pressing portion of the tubular member and is disposed along the outer surface of the bottom wall of the tubular member and the outer surface of the pressing portion of the tubular member.

11. The aerosol generation system according to claim 10, wherein the tubular member includes the pressing portion that includes two or more pressing portions, and
the heater is bent along a boundary portion between the outer surface of the bottom wall of the tubular member and the outer surface of each of the two or more pressing portions of the tubular member and is disposed along the outer surface of the bottom wall of the tubular member and the outer surface of each of the two pressing portions of the tubular member.

12. The aerosol generation system according to any one of claims 9 to 11, wherein the heater has a heat-producing region and a non-heat-producing region, and
the heater is bent at the non-heat-producing region.

13. The aerosol generation system according to any one of claims 1 to 12, wherein
the heater is constituted by a conductive track that is disposed on an electrically insulating substrate that has a flat shape,
the heater has a heat-producing region and a non-heat-producing region, and
electric resistance of the conductive track disposed in the heat-producing region is higher than electric resistance of the conductive track disposed in the non-heat-producing region.

14. The aerosol generation system according to claim 13, wherein
the non-heat-producing region of the heater is disposed at the bottom wall of the tubular member and at a side of a side wall of the tubular member, the side being close to the bottom wall, and
the heat-producing region of the heater is disposed at a side of the side wall of the tubular member, the side being close to the opening.

15. The aerosol generation system according to claim 13 or 14, wherein
the conductive track constitutes a parallel circuit in the heat-producing region of the heater.

16. The aerosol generation system according to any one of claims 13 to 15, wherein
the conductive track turns back at an end portion of the heat-producing region of the heater, the end portion being on a side far from the non-heat-producing region.

17. The aerosol generation system according to any one of claims 1 to 16, wherein
the heater is constituted by a conductive track that is disposed on an electrically insulating substrate that has a flat shape,
the heater has a heat-producing region and a non-heat-producing region,
the conductive track disposed in the heat-producing region is made of SUS,
the conductive track disposed in the non-heat-producing region is made of a material that contains at least either one of copper and nickel, and
the electrically insulating substrate is made of polyimide.

18. The aerosol generation system according to any one of claims 1 to 17, wherein
the aerosol generation system further comprises a heat transfer layer that has predetermined thermal conductivity, and
the heat transfer layer is wound to cover at least partially the tubular member and the heater that is disposed along an outer surface of a side wall of the tubular member.

19. The aerosol generation system according to claim 18, wherein
the heat transfer layer is made of graphite.

20. The aerosol generation system according to any one of claims 1 to 19, wherein
the aerosol generation system further comprises the aerosol-source material.
